# FASCICULE DE BREVET EUROPEEN

(11) **EP 4 531 652 B1**
(45) Date de publication et mention de la délivrance du brevet: **04.03.2026**
(21) Numéro de dépôt: 23730862.2
(22) Date de dépôt: 01.06.2023
(51) Int. Cl.: A61B 3/15

(54) **DISPOSITIF POUR L'EXAMEN DE LA PARTIE ANTERIEURE DE L'OEIL PAR RETRO-ILLUMINATION DE LADITE PARTIE ANTERIEURE DE L'OEIL**
VORRICHTUNG ZUR UNTERSUCHUNG DES VORDEREN AUGENTEILS DURCH RETROBELEUCHTUNG DES VORDEREN AUGENTEILS
DEVICE FOR EXAMINING THE ANTERIOR PART OF THE EYE BY RETRO-ILLUMINATION OF SAID ANTERIOR PART OF THE EYE

(30) Priorité: 03.06.2022 FR 2205394
(43) Date de publication de la demande: 09.04.2025
(73) Titulaire: Université Jean Monnet Saint-Étienne, 42100 Saint-Étienne (FR); Université Paris-Saclay, 91190 Gif-sur-Yvette (FR); Centre Hospitalier Universitaire de Saint-Etienne, 42100 Saint Etienne (FR); Centre National de la Recherche Scientifique (CNRS), 75016 Paris (FR); Institut d'Optique, 91120 Palaiseau (FR)
(72) Inventeur: GAIN, Philippe, 69009 LYON (FR); AIN, Anthony, 42000 SAINT-ETIENNE (FR); THURET, Gilles, 42330 SAINT-BONNET-LES-OULES (FR); LEPINE, Thierry, 42570 SAINT-HEAND (FR)
(74) Mandataire: Regimbeau
(86) Numéro de dépôt international: PCT/FR2023/050769
(87) Numéro de publication internationale: WO 2023/233111

(56) Documents cités:
- EP-A1- 2 415 393
- US-A- 5 202 708
- US-A- 5 695 492
- US-A1- 2018 242 846

## Description

### Domaine technique et Arrière-plan technologique

L'invention a trait au domaine de l'ophtalmologie. Plus particulièrement, l'invention concerne un dispositif pour la mise en œuvre d'un examen de l'œil, plus précisément de la partie antérieure de l'œil comprenant le segment antérieur de l'œil, par rétro-illumination, qui consiste à observer les tissus (cornée, iris, cristallin) éclairés par la lumière rétro diffusée par l'épithélium pigmenté sous la rétine (qui engendre le « reflet rouge » dans la pupille sur les photos réalisées avec flash), la lumière provenant d'une projection de lumière sur l'œil depuis une source lumineuse. Ce type de dispositif permet d'accéder notamment à une imagerie du segment antérieur de l'œil.

On entend dans la présente demande : par partie antérieure de l'œil, à la fois la zone qui va de la face avant de la cornée à la face arrière du cristallin (communément appelé segment antérieur), mais également le corps vitré se trouvant à l'arrière du cristallin et en **amont** de la rétine. En effet, par rétro-illumination, il est possible d'obtenir une image de corps flottants qui se situent en arrière du cristallin, dans le corps vitré. On peut également considérer que la partie antérieure de l'œil examiné correspond à la tunique externe l'œil (zone partant de la face avant de la cornée jusqu'à la face arrière du cristallin) et à la tunique moyenne (corps vitré).

Les ophtalmologistes utilisent couramment la «rétro-illumination» dans deux types de situation.

Tout d'abord, la rétro-illumination est utilisée lors d'un examen réalisé avec un biomicroscope ou encore appelé lampe à fente qui permet d'observer en direct le segment antérieur de l'œil. Le biomicroscope (ou lampe à fente) est un microscope double (binoculaire) dont on peut faire bouger la source lumineuse et qui permet l'examen des yeux, par l'inspection de la surface oculaire à savoir, la conjonctive, la cornée mais également les structure internes de l'œil comme le cristallin, la rétine au fond d'œil. L'œil humain est un organe facilement accessible, mais dont la taille (23 mm de diamètre) rend difficile son étude précise à l'œil nu. Certains détails de la cornée, de l'iris, du cristallin sont de l'ordre de quelques dizaines de microns. En ophtalmologie, l'examen du segment antérieur de l'œil est principalement effectué afin de dépister d'éventuelles anomalies de la surface oculaire.

Dans le cadre d'une utilisation de lampe à fente, du fait de la lumière projetée sur l'œil du patient, il y a constamment des reflets sur la cornée, et parfois sur le cristallin également. Cependant lors de l'examen, le praticien déplace manuellement la source lumineuse, ce déplacement permet de décaler le reflet gênant et donc de pouvoir observer l'ensemble de la surface oculaire. Mais si on utilise cette lampe à fente pour la prise de photo, la présence des reflets ne permet jamais l'observation de la totalité de la cornée ou du cristallin en un seul cliché.

Or, une image numérique et bien résolue de la cornée, de l'iris ou du cristallin en rétro-illumination et sans reflet est indispensable pour réaliser des analyses d'images permettant de quantifier le stade de certaines pathologies.

L'article de Timothy D. Weber et Jerome Mertz "In vivo comeal and lenticular microscopy with asymmetric fundus retroillumination" décrit un procédé de rétro-illumination dans lequel, afin d'améliorer le contraste phase-gradient, une illumination asymétrique est utilisée en illuminant un côté du fond d'œil et en imageant au moyen d'un objectif de microscope, permettant d'obtenir des images avec une résolution latérale de l'ordre du micromètre selon un champ de vue de 1 mm de diagonale dans la cornée centrale.

Si cette technique permet d'obtenir des images bien résolues, elle est restreinte à la cornée centrale et ne permet pas d'imager le segment antérieur de l'œil, qui est bien plus étendu que l'ouverture offerte par la seule cornée centrale le long d'une diagonale de 1 mm, mais s'étend de plusieurs millimètres selon l'ouverture de la pupille. Or, il peut être particulièrement avantageux d'imager le segment antérieur de l'œil, incluant des zones de la cornée excentrées par rapport à la cornée centrale. Il a en effet été découvert que des stades précoces de certaines maladies, et notamment des formes de la maladie de Fuchs, se traduisent par des défauts apparaissant dans une zone excentrée de la cornée, par exemple en face de l'interface iris-pupille, avant de migrer vers la partie centrale. Un procédé qui ne permet d'imager que la seule cornée centrale ne permet donc pas de détecter ces stades précoces. Le procédé proposé par cet article, nécessitant d'entourer la zone imagée par un anneau lumineux non imagé, ne permet pas d'imager des zones excentrées de la cornée, car l'iris fait obstacle à l'illumination.

Le deuxième type d'utilisation survient avec un microscope opératoire, pour la chirurgie de la cataracte. Cette chirurgie est la chirurgie n°1 au monde et représente 25 à 30 millions d'interventions par an, dont environ 1 million par an en France. Le reflet rouge obtenu par la rétro illumination permet de très bien voir le cristallin à opérer. Cependant, les reflets de la source lumineuse sur la cornée sont gênants car ils éblouissent l'opérateur et masquent certaines zones.

Afin d'éliminer les reflets parasites, on a proposé dans EP-A-3336597 un dispositif du type comprenant une source lumineuse d'illumination, un capteur d'image et deux sous-ensembles de polarisation complémentaires pour réduire ou éliminer les reflets. Le premier sous-ensemble de polarisation polarise la lumière d'illumination dirigée sur l'objet observé. Dans le reflet spéculaire, la polarisation de la lumière d'illumination est maintenue et ainsi, la lumière réfléchie par la surface de l'œil est filtrée par le second sous-ensemble de polarisation présent dans la voie d'observation. La lumière d'illumination qui est réfractée jusqu'à la rétine est rétro diffusée et dépolarisée par la rétine. De plus, la lumière réfléchie par la cornée conserve sa polarisation. Cependant la cornée étant composée de cinq couches, à chaque interface une partie de la lumière subit une réflexion. Plus la lumière traverse de couches plus sa polarisation va légèrement changer comparé à l'initiale créant une réflexion diffuse. En conséquence, le second sous-ensemble de polarisation va filtrer dans la lumière observée la lumière « réfléchie » présentant la polarisation « initiale » pour ne garder que la lumière rétro diffusée qui est dépolarisée. On élimine ainsi une grande partie des reflets spéculaires. Cependant dans la lumière observée, une partie est générée par la réflexion diffuse et possède une polarisation aléatoire lui permettant ainsi de passer au moins en partie à travers le second sous-ensemble de polarisation. Un tel dispositif permet donc d'atténuer les reflets spéculaires mais ne permet pas de les éliminer totalement, en particulier ceux liés à la réflexion diffuse. De plus, comme les voies d'illumination et d'observation sont confondues et prévoient un séparateur de faisceaux, on perd en intensité lumineuse pour l'observation. Pour compenser, on peut prévoir un séparateur de faisceaux polarisant. Un tel dispositif est donc complexe à réaliser et onéreux et en outre ne permet pas de supprimer tous les reflets.

EP 2 415 393 A1 divulgue un dispositif pour l'examen de la partie antérieure de l'œil par rétro-illumination comprenant des moyens d'illumination avec une source de lumière (source 1, alinéa 28), des moyens d'imagerie (dispositif d'imagerie 35, alinéa 28), et des moyens de modulation (prisme triangulaire 7, alinéa 24) pour modifier la longueur du chemin optique.

### Présentation de l'invention

La présente invention a donc pour objet principal de proposer un dispositif permettant un examen et notamment une prise d'image du segment antérieur avec une structure moins complexe tout en offrant une prise de vue dans laquelle au moins les reflets parasites intrinsèques au système sont supprimés.

La présente invention a pour but de proposer un dispositif pour l'examen de la partie antérieure de l'œil par rétro-illumination de ladite partie antérieure de l'œil, comportant des moyens d'illumination de l'œil comprenant une source de lumière et configurés pour émettre un faisceau lumineux, et des moyens d'imagerie de la partie antérieure de l'œil comprenant un capteur optique configuré pour capturer une image du segment antérieur de l'œil, comprenant en outre des moyens de modulation configurés pour éteindre au moins une zone du faisceau lumineux émis depuis la source lumineuse et pour créer ainsi au moins une zone non illuminée (ZNI) à la surface de l'œil.

Les moyens de modulation peuvent comprendre un obturateur placé dans le faisceau lumineux pour en bloquer des rayons, éteignant ainsi une zone du faisceau lumineux. Les moyens de modulation peuvent également être

De manière avantageuse, les moyens de modulation d'une ou plusieurs parties du faisceau lumineux envoyé vers l'œil du patient permettent d'éclairer l'œil de manière modulable.

De préférence, cette illumination de l'œil peut être sélective, en évitant notamment d'éclairer directement une ou des zones de l'œil responsables des reflets spéculaires classiques. Les moyens de modulation permettent ainsi de créer des zones non illuminées dites « éteintes » à la surface de l'œil à examiner la modulation consistant à supprimer une ou plusieurs parties du faisceau lumineux, qui forment ces zones « éteintes » n'engendrant donc plus de reflets lors de la prise d'image. Cette suppression d'une ou plusieurs parties du faisceau lumineux peut s'effectuer par des moyens d'obturation interposés sur le passage ou par des moyens de non réflexion d'une ou plusieurs parties du faisceau.

Les zones responsables des reflets spéculaires classiques ne sont donc plus éclairées directement par la source lumineuse du dispositif selon l'invention mais seulement par la rétrodiffusion de la lumière par la rétine ce qui rend l'image homogène et sans reflet.

De préférence, les moyens d'imagerie sont configurés pour que le capteur d'image comprenne dans son champ de vision la zone non illuminée et au moins une zone illuminée par le faisceau lumineux à la surface de l'œil. Seule les zones responsables des reflets spéculaires ne sont pas éclairées, ce qui permet donc d'éclairer les autres zones, introduisant suffisamment de lumière dans l'œil pour une bonne rétrodiffusion. En revanche, l'ensemble des zones, illuminées ou non par le faisceau lumineux sont dans le champ de vision, afin de maximiser l'étendue des tissus imagés dans le segment antérieur de l'œil, et en particulier pour les parties excentrées de la cornée.

Typiquement, les moyens d'imagerie sont configurés pour présenter un champ de vision couvrant une surface d'un diamètre (plus grande dimension) supérieur ou égal à 4 mm à la surface de l'oeil, et de préférence encore supérieur à 5 mm. De préférence, une zone non illuminée s'étend sur une superficie d'un diamètre (plus grande dimension) inférieur ou égal à 3 mm, et de préférence inférieur ou égal à 2 mm.

De préférence, les moyens de modulation sont configurés pour qu'une zone éteinte soit entourée par une zone non éteinte dans le faisceau lumineux. Ainsi, une zone non illuminée par le faisceau lumineux à la surface de l'œil est entourée par une zone illuminée par le faisceau lumineux à la surface de l'œil, ce qui permet de limiter l'inhomogénéité de l'éclairage par rétrodiffusion. De préférence encore, une zone éteinte est au centre du faisceau lumineux, et la zone non illuminée est au centre de la cornée.

Les moyens d'imagerie comprennent un appareil de capture d'image. De préférence, les moyens d'imagerie comprennent un bloc optique, des moyens de polarisation et un appareil de capture d'image. L'image de l'œil peut être ainsi capturée sur un capteur optique qui photographie le plan d'imagerie au niveau de l'œil. Dans les moyens d'imagerie, on prévoit des moyens de polarisation tels qu'un analyseur (polariseur) qui éteignent les reflets secondaires liés à la partie réfléchie par la cornée et le cristallin du faisceau lumineux projeté. Le faisceau lumineux constitué de la partie réfractée dans l'œil jusqu' à la rétine est rétrodiffusé et dépolarisé par la rétine, ce qui permet d'éclairer et d'observer les tissus (cornée, iris, cristallin) éclairés par cette lumière rétro diffusée par l'épithélium pigmenté sous la rétine, les zones éteintes étant ainsi éclairées par ladite lumière rétrodiffusée. L'analyseur peut ensuite polariser le faisceau lumineux émis par l'œil tout en éteignant, c'est-à-dire en supprimant des reflets parasites intrinsèques au système et les faibles reflets secondaires possibles de la surface cornéenne.

. On obtient ainsi une imagerie améliorée du segment antérieur de l'œil à examiner grâce à la rétro-illumination provenant de la rétine qui ne nécessite pas de traitement supplémentaire de l'image visant à éliminer les reflets.

En variante, les moyens de modulation d'une partie du faisceau lumineux émis par la source d'illumination suppriment une partie du faisceau lumineux et autorisent dans cette zone « éteinte » l'émission d'un faisceau lumineux provenant d'une source lumineuse présentant une longueur d'onde différente, permettant de créer des zones de l'œil illuminées différemment, tel que pour créer un faisceau dit de fixation aidant à l'examen. Les moyens d'imagerie comportent alors des moyens de blocage dudit faisceau lumineux de fixation réfléchi par l'œil tel qu'un filtre spectral, le filtre spectral permettant de bloquer en fonction de sa longueur d'onde le faisceau lumineux de fixation réfléchi et rétrodiffusé.

Le dispositif permet avantageusement une prise d'image du segment antérieur tout en offrant une prise de vue dans laquelle les reflets parasites intrinsèques au système et également les reflets secondaires possibles de la surface cornéenne et du cristallin naturel ou artificiel sont supprimés.

Un dispositif selon l'invention permet ainsi d'établir des diagnostics et des suivis de pathologies fréquentes comme par exemple la dystrophie endothéliale cornéenne de Fuchs, encore appelée Cornea Guttata à son stade précoce, qui affecte 4 à 10% des adultes de plus de 40 ans, ou la cataracte secondaire (opacification apparaissant derrière un implant de chirurgie de cataracte) qui concerne environ 20% des opérés. En effet, le terme « Cornea Guttata » désigne la présence d'anomalies de la face postérieure de la cornée sous forme de "gouttes" microscopiques visible avec le dispositif de rétroillumination selon l'invention, ces "gouttes" étant le plus souvent le signe du début d'une dystrophie endothéliale cornéenne de Fuchs.

Le dispositif selon l'invention peut avantageusement servir à imager de façon fiable la cataracte pour en évaluer sa densité et rendre objectif le diagnostic.

Le dispositif selon l'invention permet de rendre possible l'analyse d'image (quantification de certaines éléments caractéristiques des pathologies visées) de ces images en rétro-illumination pour effectuer un diagnostic de sévérité de certaines pathologies et suivre leur évolution dans le temps (vitesse d'aggravation, aide à la décision médicale). Un tel dispositif selon l'invention est bien plus avantageux que les dispositifs actuels de diagnostic qui se basent sur une photo partielle (en excluant les zones de reflets de l'analyse) ou sur au moins deux prises de photos puis reconstruction d'images.

De préférence, les moyens d'illumination comprennent des moyens de réflexion constitués d'un miroir plan dit miroir de réflexion pour projeter le faisceau lumineux d'illumination vers l'œil à examiner, ce qui permet de conserver l'intensité du faisceau lumineux créé. En conséquence, la voie d'illumination définie par ces moyens d'illumination est distincte de la voie d'imagerie définie par les moyens d'imagerie. Les deux voies d'illumination et d'imagerie n'ayant pas d'interface commune, leur structure et mise en œuvre sont donc plus modulables. Il est alors possible de prévoir que les moyens d'illumination et des moyens d'imagerie, sont sous forme de deux modules distincts. Il est ainsi notamment possible de décentrer la voie d'imagerie par rapport à la voie d'illumination, les axes optiques d'illumination et d'imagerie étant décalés. De plus, il est ainsi possible d'adapter ces deux voies sur des lampes à fente du commerce existante. On peut prévoir également que les axes optiques d'illumination et d'imagerie sont confondus.

Le décalage entre l'axe optique d'illumination et l'axe optique d'imagerie peut permettre de mieux mettre en évidence des détails de l'œil. Il est par exemple possible de prévoir un décalage angulaire compris entre 3° et 15°, et de préférence entre 5° et 10° entre l'axe optique d'illumination et l'axe optique d'imagerie. Un angle trop important fait courir le risque, surtout avec une image grand champ, d'une inhomogénéité d'éclairage, avec une partie de l'image moins bien éclairée. A l'inverse, avec un angle de décalage trop faible, voire nul, les structures en reliefs que l'on peut voir sur les images auront moins de contraste.

En variante, on peut prévoir dans les moyens d'illumination un cube polarisant constituant les moyens de polarisation et qui remplace également les moyens de réflexion.

L'invention a également pour objet de proposer un dispositif pour l'examen de la partie antérieure de l'œil par rétro-illumination, comportant des moyens d'illumination de l'œil comprenant une source de lumière et des moyens d'imagerie de la partie antérieure de l'œil, caractérisé en ce que les moyens d'illumination comprennent une source de lumière du type diode électroluminescente (DEL), des moyen de collimation pour former un faisceau lumineux tel qu'un bloc optique, un diaphragme, des moyens de polarisation, des moyens de réflexion pour l'envoi du faisceau lumineux vers l'œil d'un patient, et un moyen d'obturation d'une partie du faisceau lumineux formé constituant les moyens de modulation d'une zone du faisceau lumineux.

De manière avantageuse, le moyen d'obturation ou obturateur permet de créer un faisceau de lumière dont une zone est « coupée », donc éteinte par l'obturateur. Ce dernier est de préférence constitué par une forme opaque interposée sur la trajectoire du faisceau lumineux, absorbant une partie du faisceau lumineux. De préférence, l'obturateur est à l'intérieur du faisceau lumineux, c'est-à-dire disposé de sorte à être dépassé de tous côtés par une partie non éteinte du faisceau lumineux.

La lumière émise par la source est concentrée par un bloc optique constitué d'un groupe de lentilles tel qu'une lentille de collimation en tant que moyens de collimation de la lumière émise par la source et un doublet afocal formé de deux lentilles qui concentre le faisceau sur le diaphragme pour optimiser la puissance apportée, le diamètre du faisceau lumineux ainsi formé étant réduit. On peut envisager une lentille de collimation permettant de sortir le faisceau avec le bon diamètre.

L'obturateur peut alors être placé dans la trajectoire du faisceau lumineux, après le bloc optique, en amont, au centre ou en aval du diaphragme.

L'obturateur, placé au centre du faisceau lumineux formé en sortie du bloc optique, par exemple au centre du diaphragme permet d'empêcher l'illumination de la zone centrale de l'œil qui présente un reflet habituel, ledit obturateur étant dans le plan du diaphragme, tous les deux étant conjugués avec le plan d'imagerie devant l'œil.

De préférence, les moyens d'illumination du dispositif comprennent au moins un miroir plan interposé entre le diaphragme et les moyens de réflexion, renvoyant le faisceau lumineux en sortie du diaphragme vers les moyens de réflexion constitués notamment d'un miroir plan dit miroir de réflexion pour l'envoi du faisceau lumineux vers l'œil d'un patient. Les deux miroirs plans sont inclinés à 45° ce qui permet avantageusement de réduire l'encombrement du dispositif.

Le miroir de réflexion est de préférence conjugué avec le foyer image d'une lentille, positionnée entre les deux miroirs, afin de minimiser la taille de ce miroir de réflexion ce qui maximise l'intensité du faisceau lumineux reçu envoyé au patient.

L'invention a également pour objet, un dispositif pour l'examen de la partie antérieure de l'œil d'un patient par rétro-illumination, comportant des moyens d'illumination de l'œil comprenant une source de lumière et des moyens d'imagerie de la partie antérieure de l'œil, caractérisé en ce que les moyens d'illumination comprennent une source de lumière du type diode électroluminescente (DEL), des moyens de collimation pour former un faisceau lumineux, un diaphragme, des moyens de polarisation, des moyens de réflexion du faisceau lumineux vers l'œil du patient, et un miroir présentant au moins un orifice ne renvoyant pas ainsi une partie du faisceau lumineux émis depuis la source et constituant ainsi les moyens de modulation d'une zone du faisceau lumineux.

De préférence, le miroir est parabolique, constituant ainsi également les moyens de collimation, ce qui évite la présence d'un bloc optique comprenant des moyens de collimation comme décrit précédemment. On peut également prévoir un deuxième miroir après le diaphragme, également parabolique. Toutefois, on peut également envisager que les miroirs sont plans et dans cette configuration, on prévoit le bloc optique tel que précédemment décrit.

De manière avantageuse, un tel dispositif présente ainsi une structure simple et fiable quant à la formation de la zone éteinte du faisceau lumineux.

De plus, de manière préférée, les moyens d'illumination comprennent une deuxième source lumineuse, positionnée à l'arrière du premier miroir et émettant un faisceau lumineux au travers de l'orifice dudit premier miroir, le faisceau lumineux issu de cette source lumineuse étant d'une longueur d'onde différente de celle du faisceau lumineux émis par la première source lumineuse et formant un faisceau lumineux dit de fixation.

De manière avantageuse, les moyens d'imagerie comprennent alors un filtre spectral destiné à éteindre le faisceau lumineux de fixation dans l'image observée.

Le dispositif selon ce mode de réalisation de l'invention permet de proposer un examen fiable en permettant d'intégrer dans le dispositif un faisceau lumineux de fixation de manière simple permettant ainsi au patient de rester le plus immobile possible pendant l'examen, afin d'avoir une meilleure résolution d'image avec moins de flou de bougé, et donc une meilleure qualité de la prise de vue obtenue.

Un autre objet de l'invention est de proposer un dispositif dans lequel les moyens de modulation d'au moins une zone du faisceau lumineux d'illumination sont constitués d'un modulateur spatial de lumière connu sous l'acronyme anglais SLM (Spatial Light Modulator) et ses moyens de pilotage qui permet d'éliminer en temps réel les reflets spécifiques à chaque patient. On obtient ainsi une suppression des reflets active, adaptative et rapide.

Un tel modulateur SLM présente une fonction optique consistant à éteindre une ou des parties du faisceau : comme un « marche-arrêt » de pixels, et ce par polarisation, réflexion etc.

Ainsi, un modulateur SLM peut être un élément d'optique diffractive modulant la phase du faisceau lumineux à l'aide de cristaux liquides. Ce système exploite le principe d'anisotropie des cristaux liquides, c'est-à-dire la modification de l'indice des cristaux liquides, en fonction de leur orientation spatiale. L'orientation des cristaux liquide peut être réalisée à l'aide d'un champ électrique. Ainsi, en modifiant localement l'indice des cristaux liquides, il est possible de modifier le front d'onde du faisceau lumineux.

On peut également envisager que ce modulateur SLM est une matrice de micro-miroirs, connu sous l'acronyme anglais DMD ( digital micromirror device ).

Le SLM permet donc de façonner d'une manière dynamique le front d'onde du faisceau lumineux. Cette modulation permet la mise en forme du faisceau projeté d'une manière dynamique et reconfigurable. Il est ainsi possible de segmenter les reflets ce qui permet d'obtenir une image simple à traiter, les reflets se distinguant fortement du fond en terme de niveau de gris.

Il s'agit d'un éclairage asservi par traitement informatique de la voie d'imagerie. Son principe est le suivant :
L'image réalisée par la voie d'imagerie est analysée par l'ordinateur ;
L'ordinateur segmente les zones de reflets
Cette segmentation est retranscrite sur un modulateur spatial pour obturer une partie du faisceau lumineux émis et éteindre, dans la voie d'éclairage, les zones responsables des reflets,
Une « nouvelle » image du modulateur est projetée dans le plan d'imagerie, les zones responsables des reflets ne sont plus illuminées, ces étapes étant répétées en boucle pour supprimer en temps réel les reflets.
Prise d'image finale : Les reflets sont supprimés rapidement tout en s'adaptant à l'œil du patient.

Les moyens d'illumination comprennent une source de lumière tel que diode électroluminescente (DEL). Dans ce cas, le dispositif d'examen selon l'invention peut être couplé à un laser femtoseconde (YAG) afin de permettre de réaliser plus que du diagnostic (avec le dispositif selon l'invention) mais également une intervention en le couplant avec un laser pour réaliser à la fois un diagnostic et un traitement. Par conséquent, cet acte médical réalisé par des dispositifs hybrides combinant lampe à fente laser, par exemple, est bien mieux réalisé lorsque l'imagerie est sans reflet.

L'invention, aussi bien dans ces différentes formes de réalisation, offre donc un dispositif de faible encombrement, d'un coût de fabrication modéré et modulable. De plus, ce dispositif, du fait d'une meilleure qualité d'image obtenue, permet de faire du diagnostic précoce de pathologies telles que la guttata.

En outre, le dispositif selon l'invention peut faire partie d'un ensemble qui combine donc ce dispositif d'examen de la partie antérieure de l'œil par rétro-illumination de la partie antérieure de l'œil, permettant un diagnostic en retro-illumination et un dispositif de traitement par laser YAG de la cataracte secondaire ou de corps flottant du vitré ou par laser femto pour la cataracte.

### Présentation des figures

On décrira maintenant l'invention plus en détails en référence au dessin dans lequel les figures représentent :
- la figure 1 est une représentation schématique selon un premier mode de réalisation d'un dispositif de l'invention ;
- la figure 2 est une représentation schématique de la trajectoire des faisceaux lumineux d'illumination et d'imagerie dans le dispositif selon la figure 1 ;
- la figure 3 est une représentation schématique en section transversale du faisceau lumineux d'illumination et d'imagerie à différentes positions dans la trajectoire selon la figure 2 ;
- la figure 4 est une représentation schématique selon un second mode de réalisation d'un dispositif de l'invention ;
- la figure 5 est une représentation schématique de la trajectoire des faisceaux lumineux d'illumination et d'imagerie dans le dispositif selon la figure 4 ;
- la figure 6 est une représentation schématique en section transversale du faisceau lumineux d'illumination et d'imagerie à différentes positions dans la trajectoire selon la figure 5 ;
- la figure 7 est une représentation schématique selon un troisième mode de réalisation d'un dispositif selon l'invention ;
- la figure 8 est une photographie d'une cornée obtenue par un dispositif selon l'invention ;
- la figure 9 est une photographie d'un cristallin obtenu selon l'invention.

### Description détaillée

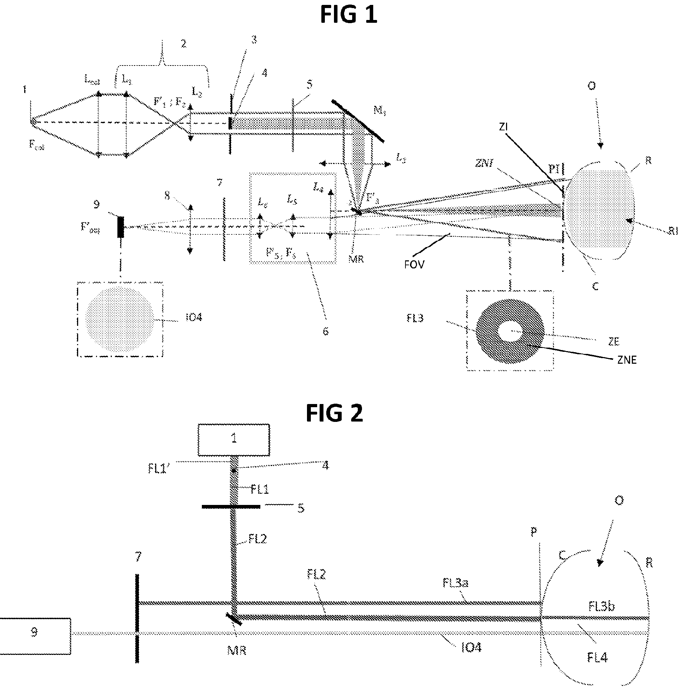

Comme on peut le voir à la figure 1, un exemple de réalisation d'un dispositif selon l'invention comporte une voie d'illumination d'un œil O d'un patient à examiner et une voie d'imagerie permettant d'observer cet œil O et en particulier de prendre des clichés du segment avant de l'œil.

La voie d'illumination est définie par des moyens d'illumination comportant une source de lumière 1 telle qu'une diode électroluminescente, connue sous l'acronyme anglais LED pour « Light Emitting Diode ». Les moyens d'illumination sont configurés pour émettre un faisceau lumineux FL1 en direction de l'œil O recouvrant de préférence le champ de vision FOV du capteur des moyens d'imagerie au niveau de la surface de l'œil O. De préférence, le faisceau lumineux FL1 s'étend, dans un plan d'incidence à l'œil O perpendiculaire à la propagation lumineuse, sur une superficie présentant un diamètre (plus grande dimension) d'au moins 4 mm, et de préférence d'au moins 5 mm. L'éclairage d'une grande superficie de l'œil O permet de faire rentrer plus de lumière dans l'œil O, permettant d'augmenter la rétrodiffusion, et améliorant l'image acquise.

La source LED 1 est choisie pour présenter un spectre d'émission dont le pic est de préférence voisin de 780 nm, correspondant à un faisceau lumineux peu absorbé par l'œil et assez proche du visible pour que le patient puisse fixer la source 1 sans être ébloui.

La source 1 est modulée en intensité par des moyens de commande de manière classique en soi. Bien entendu, le principe de la suppression de reflet peut être appliqué à l'ensemble du spectre de la lumière : toute autre longueur d'onde pourrait être utilisée.

Les moyens d'illumination comportent ensuite un bloc optique constitué d'une lentille Lcol permettant de collimater la lumière émise par la source 1 vers un doublet afocal 2 formé de deux lentilles L1 et L2 dont les foyers F'1 et F2 sont confondus.

Ce doublet afocal 2 permet ainsi de concentrer le faisceau lumineux FL1 émis par la source 1 sur un diaphragme 3 pour optimiser la puissance apportée, le diamètre du faisceau FL1 étant ainsi réduit.

Le dispositif selon l'invention comporte des moyens de modulation d'une zone ou partie du faisceau lumineux FL1, permettant de créer un faisceau lumineux sur l'œil d'un patient en générant une zone dite « éteinte » dans le faisceau lumineux FL1 et créer ainsi au moins une zone non illuminée ZNI à la surface de l'œil, tandis que les zones non éteintes du faisceau lumineux FL1 créent au moins une zone illuminée ZI à la surface de l'œil.

Dans l'exemple représenté à la figure 1, les moyens de modulation du faisceau lumineux sont constitués d'un obturateur 4, ici positionné dans le plan du diaphragme 3. De préférence l'obturateur 4 est constitué d'une forme en matériau opaque, de préférence avec une surface obturante continue, comme par exemple un disque opaque. L'obturateur 4 peut par exemple être maintenu par des éléments de support en matériau transparent sans incidence sur le faisceau lumineux FL1 passant, par exemple dans le plan du diaphragme 3.

Ce faisceau lumineux FL1 est ensuite polarisé par un polariseur 5 sous forme d'un faisceau lumineux FL2.

Afin de limiter l'encombrement du dispositif, on prévoit de préférence deux miroirs plans M1 et MR. Le miroir MR constitue les moyens de réflexion du faisceau lumineux vers l'œil du patient et le miroir M1 qui peut être optionnel, permet de limiter l'encombrement des moyens d'éclairage.

Le miroir MR est associé avec le foyer image d'une troisième lentille L3 pour minimiser la taille du miroir MR, ce qui maximise le faisceau lumineux FL2 reçu et envoyé vers l'œil du patient sous forme d'un faisceau lumineux FL3, de préférence divergent.

Ce faisceau lumineux FL3 envoyé vers le patient traverse, pour une partie dudit faisceau lumineux FL3, la cornée C sous forme d'un faisceau lumineux FL3b jusqu'à la rétine R sur laquelle ce faisceau lumineux FL3b est rétro-diffusé, dépolarisé sous forme d'un faisceau lumineux FL4 ce qui illumine le plan d'imagerie PI situé à l'avant de la cornée C générant la rétro illumination du segment antérieur et du corps vitré de l'œil. Une partie du faisceau lumineux FL3 est également réfléchi par la cornée C sous la forme d'un faisceau lumineux FL3a.

Ce faisceau réfléchi FL3a et l'image de l'œil IO4 entrent ensuite dans la voie d'imagerie. La figure 2 représente l'ensemble de ces faisceaux lumineux et leurs trajectoires.

Comme on peut le voir, l'obturateur 4 interrompt le faisceau lumineux FL1', de manière centrale, de ce fait, on peut considérer que le faisceau lumineux FL1 en sortie du diaphragme 3 présente une section tubulaire présentant une zone centrale éteinte ZE et une zone périphérique non éteinte ZNE. La zone éteinte conduit à empêcher l'illumination d'une zone de la cornée C en correspondance de l'obturateur 4, comme on peut le voir sur la figure 1. Cette zone non directement illuminée ZNI, de la cornée C n'émet plus de reflets parasites empêchant l'examen de la zone antérieure de l'œil par la rétro-illumination. Cette zone de la cornée non illuminée ZNI correspond à une zone dans laquelle des reflets apparaissent systématiquement. En revanche, une autre zone de la cornée C reçoit la lumière du faisceau lumineux non éteinte par l'obturateur 4, et forme une zone directement illuminée ZI par le faisceau lumineux. Les moyens d'imagerie sont configurés pour que le capteur d'image comprenne dans son champ de vision FOV la zone non illuminée ZNI et au moins une zone illuminée ZI par le faisceau lumineux à la surface de l'œil.

L'image de l'œil IO4 est alors captée par la voie d'imagerie définie par les moyens d'imagerie et notamment par le bloc optique 6 comprenant une lentille L4 permettant de capter de manière décalée par rapport l'axe optique d'illumination, cette image IO4 rejetée à l'infini par L4 puis de projeter vers l'infini cette image IO4 à l'aide de deux lentilles L5 et L6 formant un doublet afocal, vers un capteur optique 9. Ce bloc optique 6 correspond à une tête optique classiquement utilisée dans les lampes à fente. Une tête optique de ce type permet d'alterner entre cinq grossissements différents (x6 x10 x16 x25 x40).

En sortie de cette tête optique 6, l'image est rejetée à l'infini et on passe d'un axe optique principal à deux axes optiques comme cela est prévu pour des oculaires de lampe à fente. Une telle tête optique 6 conçue pour l'imagerie du segment antérieur de l'œil, respecte les qualités optiques nécessaires pour imager la cornée et le cristallin. Cette tête optique 6 peut être remplacée par tout bloc optique ou tout tête optique respectant les caractéristiques voulues.

L'image de l'œil IO4 dépolarisée par la rétine est ensuite traité par des moyens de polarisation tels qu'un analyseur (c'est-à-dire un polariseur 7) puis au travers de l'objectif (lentille L8) d'un appareil de capture d'image, projeté vers le foyer de l'appareil où se trouve le capteur optique approprié 9.

Le polariseur 7 est linéaire, de préférence vissé sur l'objectif 8 et est utilisé avec un axe croisé à 90° à l'axe de polarisation de la lumière induit par le polariseur 5 des moyens d'illumination afin de supprimer les reflets parasites internes du système optiques ainsi qu'une partie des reflets parasites, tels que la partie du faisceau lumineux FL3 réfléchi par la cornée C sous la forme d'un faisceau lumineux FL3a qui a gardé la même polarisation que FL2.

Dans l'exemple de mise en œuvre représenté, on utilise un capteur optique 9 qui transforme des photons en charge électrique par effet photoélectrique, tel qu'un capteur à semi-conducteur à oxyde de métal complémentaire connu sous l'acronyme anglais CMOS (Complementary Metal-Oxide Semiconductor) ou un capteur à transfert de charge connu sous l'acronyme anglais CCD (Charge-Coupled Device).

On choisit de préférence un capteur 9 dont le temps de pose est de préférence réglable entre 1 µs à 1 minute, ce qui couvre largement le temps de pose pour réduire le flou de bougé et qui présente une résolution de 12 Mégapixels (3000x4096), soit une surface carrée utile de 9 Mégapixels pour imager la cornée. Un capteur de ce type peut être celui proposé sous la référence commerciale VCXU-123M. Tout autre type de capteur présentant des caractéristiques appropriées peut être utilisé.

Au capteur optique 9 est associé un logiciel permettant d'enregistrer les images qui, comme on l'a vu n'ont pas besoin de traitement d'images et sont utilisables brutes. On peut toutefois prévoir des moyens de traitement de ces images si nécessaires.

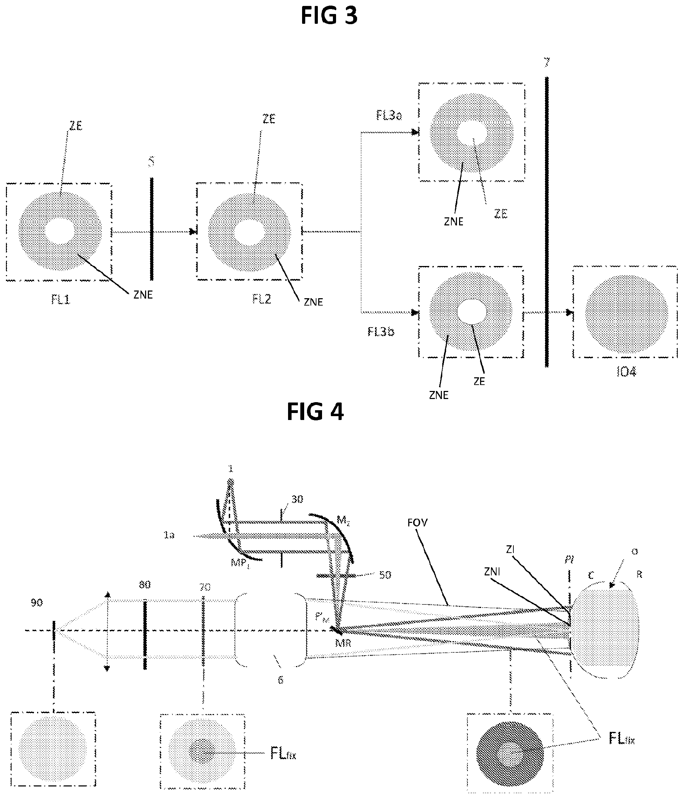

Comme on peut le voir sur la figure 3 qui représente les faisceaux lumineux présents à différentes étapes de la trajectoire entre la source 1 et le capteur optique 9, l'image de l'œil IO4 est de nouveau une image lumineuse complète correspondant à la rétro-illumination par la rétine R, le faisceau lumineux FL3a est lui éteint par l'analyseur 7.

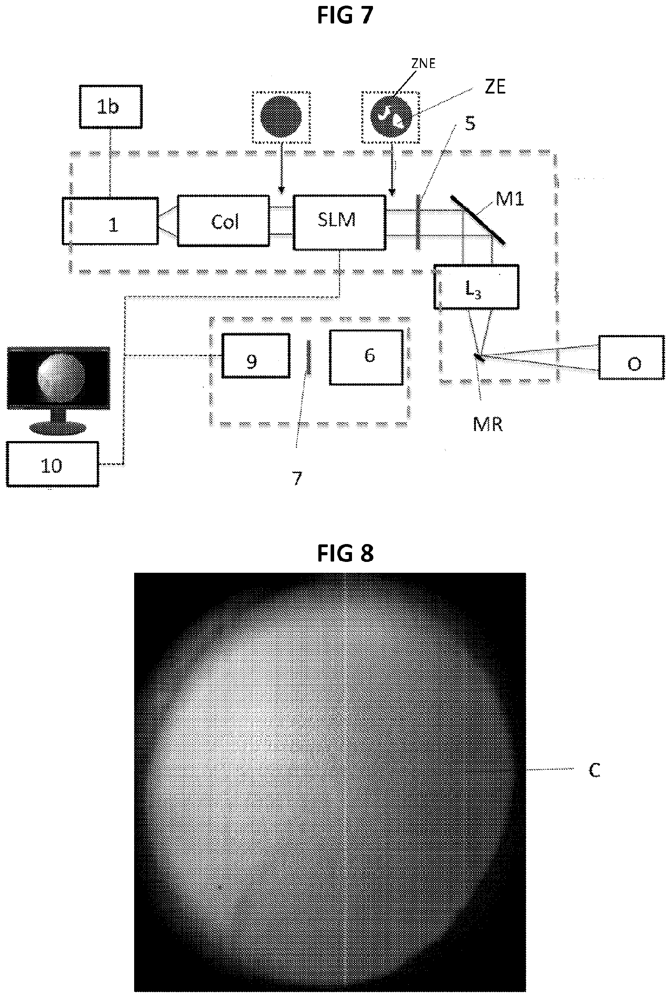

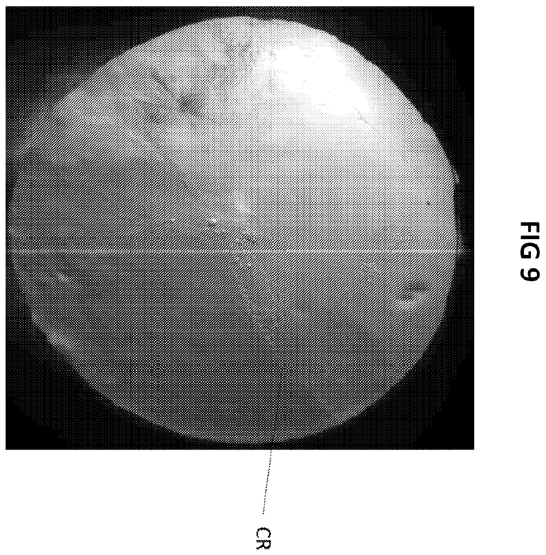

La figure 8 représente une photographie de la partie antérieure, cornée C, d'un œil examiné par un dispositif selon l'invention, où l'on peut voir qu'il n'y a plus de reflet tandis que la figure 9 représente une photographie d'un cristallin CR.

Dans un second exemple de réalisation de l'invention représenté aux figures 4, 5 et 6, on propose une variante de la voie d'illumination qui comporte deux sources lumineuses émettant dans des longueurs d'ondes différentes et distinctes. Par exemple, une première source lumineuse 1 émetun premier faisceau lumineux possédant une longueur d'onde de 780 nm et une seconde source 1a émet un second faisceau lumineux possédant une longueur d'onde de 550 nm. Cette seconde source 1a forme un faisceau lumineux différent dit de fixation FL_{fix} permettant au patient en fixant ce faisceau lumineux FL_{fix} de rester immobile lors de l'examen.

Les moyens d'illumination dans ce mode de réalisation comprennent un miroir MP1, de préférence parabolique, qui présente un trou central qui ne renvoie pas le faisceau FL1 et forme au sein de ce faisceau F1 une zone éteinte ZE, et renvoie une partie de la lumière formant la zone non éteinte (ZNE) du faisceau lumineux. Le miroir parabolique MP1 constitue les moyens de modulation d'une zone du faisceau lumineux et permet aussi de collimater le faisceau lumineux F1. Ce miroir MP1 a donc ainsi une double fonction : optique et modulation. Cette zone éteinte du faisceau lumineux FL1 est occupée par le faisceau lumineux de fixation FL_{fix} émis par la source de lumière 1a.

Ainsi, au travers de l'orifice central du miroir MP1 passe le faisceau lumineux de fixation FL_{fix} émis par la seconde source 1a, créant ainsi un faisceau lumineux FL10 issu des deux sources 1 et 1a.

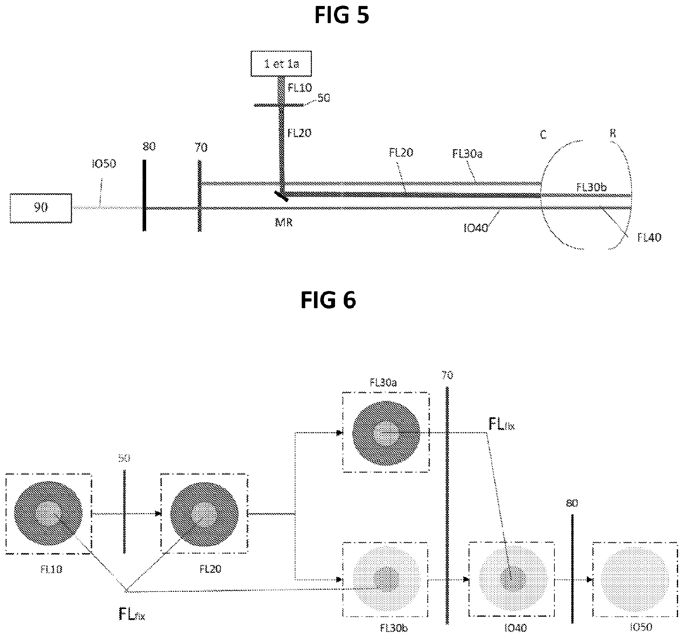

Le faisceau lumineux FL1 émis par la source 1 est donc renvoyé à 90° par un miroir parabolique MP1 sous forme d'un faisceau FL10 qui intègre, dans sa zone éteinte, le faisceau lumineux de fixation FL_{fix}. Ce faisceau FL10 traverse un diaphragme 30 pour venir sur un second miroir parabolique MP2 sur lequel il est renvoyé à 90° au travers d'un polariseur 50 sous forme d'un faisceau lumineux FL20 vers un miroir plan de réflexion MR qui le renvoie ensuite vers l'œil à examiner. Comme on peut le voir sur la figure 4 et à la figure 6, le faisceau lumineux FL20 arrivant sur la cornée C est constitué du faisceau lumineux FL1 avec sa zone éteinte ZE et sa zone non éteinte ZNE, et du faisceau lumineux de fixation FL_{fix}, confondus et polarisés. Le miroir parabolique MP2 a la fonction complémentaire de focaliser le faisceau (en plus de renvoyer à 90°).

Le faisceau lumineux FL20 est en partie réfléchi par l'œil sous forme d'un faisceau lumineux FL30a tandis qu'une autre partie FL30b traverse la cornée et est rétrodiffusée et dépolarisée par la rétine R sous forme d'un faisceau lumineux FL40. Ce dernier permet d'illuminer par rétro-illumination le segment antérieur de l'œil. L'image de l'œil IO40 est alors captée par la voie d'imagerie, au niveau d'une tête optique 60 tel que précédemment décrit, puis d'un polariseur/analyseur 70 ainsi qu'un filtre spectral 80 approprié pour bloquer le faisceau lumineux réfléchie FL30a et laisser passer l'IO40 tout en filtrant la partie issue du faisceau lumineux de fixation. En filtrant cette partie « fixation » de l'IO30, on élimine le faisceau de fixation et les reflets liés à cette zone centrale et on obtient une image de l'œil IO50 sans reflet parasite.

Comme on peut le voir à la figure 7, un dispositif selon l'invention est décrit selon un troisième mode de réalisation dans lequel la voie d'illumination comprend une source lumineuse 1, par exemple à DEL, gérée par des moyens de commande 1b, un collimateur Col, un modulateur spatial de lumière SLM, un polariseur 5, un premier miroir M1 une lentille L3, puis le miroir MR de réflexion du faisceau lumineux vers l'œil du patient O.

La voie d'imagerie comprend un bloc optique 6, un analyseur 7 et un capteur optique 9. A ce capteur optique 9 sont associés des moyens de traitement informatique (logiciel de pilotage) qui permettent d'éliminer en temps réel les reflets spécifiques présents à la surface de l'image prise et ce pour chaque patient. On obtient ainsi une suppression des reflets adaptative et rapide. Les moyens de traitement informatique permettent alors de piloter les moyens d'illumination, l'éclairage étant asservi par le traitement informatique de la voie d'imagerie.

Le principe est le suivant : l'image réalisée par la voie d'imagerie est analysée par un ordinateur 10 qui segmente les zones de reflets décelées et cette segmentation est retranscrite sur le modulateur spatial SLM pour éteindre, dans la voie d'éclairage, les zones responsables des reflets. Une « nouvelle » image du modulateur est projetée dans le plan d'imagerie, les zones responsables des reflets ne sont plus illuminées ZNI tandis que demeurent les autres zones directement illuminées ZI, ces étapes étant répétées en boucle pour supprimer en temps réel les reflets. Les reflets sont donc tous supprimés rapidement tout en s'adaptant à l'œil du patient, grâce à l'extinction de zones du faisceau lumineux.

Dans les différents modes de réalisation décrits, les moyens de modulation sont configurés pour éteindre au moins une zone ZE du faisceau lumineux FL1 émis depuis la source lumineuse 1 et pour créer ainsi au moins une zone non illuminée ZNI à la surface de l'œil O. Le faisceau lumineux FL1 comprend alors au moins une zone éteinte ZE et au moins une zone non éteinte ZNE, la zone non éteinte ZNE créant une zone illuminée ZI à la surface de l'œil O.

Les moyens d'imagerie sont configurés pour que le capteur d'image comprenne dans son champ de vision FOV la zone non illuminée ZNI et au moins une zone illuminée ZI par le faisceau lumineux à la surface de l'œil O. Le champ de vision FOV, ou champ de vue, également connu sous le terme anglais de « field of view », est l'angle solide au travers duquel le capteur est sensible à la lumière en provenance de l'œil O, à travers les différents composants (lentilles, etc.) de la voie d'imagerie.

Ainsi, l'image acquise par le capteur des moyens d'imagerie représente à la fois des tissus en correspondance avec la zone non illuminée ZNI et des tissus en correspondance avec la zone non illuminée ZNI. Par exemple, dans le cas de l'utilisation d'un obturateur 4 central, l'image peut montrer le centre de l'axe optique d'imagerie correspondant à une zone non illuminée ZNI, mais également des parties du segment antérieur de l'œil O excentrées, et correspondant à la zone illuminée ZI entourant la zone non illuminée ZNI sur la cornée C.

Puisque le champ de vision FOV inclut une zone illuminée ZI par le faisceau lumineux, l'utilisation d'un analyseur est d'autant plus utile pour supprimer les reflets secondaires liés à la partie réfléchie par la cornée.

De préférence, lequel les moyens d'imagerie sont configurés pour présenter un champ de vision FOV couvrant une surface d'un diamètre supérieur ou égal à 4 mm sur l'œil O (par exemple dans un plan d'imagerie), et de préférence supérieur ou égal à 5 mm. Un tel champ de vision FOV permet d'imager le segment antérieur de l'œil O de sorte à permettre de rendre visible des tissus qui ne sont pas situés à proximité immédiate de l'axe optique d'imagerie. En outre, il est alors possible d'imager une plus grande surface du segment antérieur de l'œil O sans avoir besoin de réitérer une acquisition d'image, et donc le risque de mouvement de l'œil O.

Les moyens de modulation sont configurés de sorte que les zones éteintes ZE du faisceau lumineux représentent moins de 50% de la superficie des zones non éteintes ZNE dans une section du faisceau lumineux, mais de préférence représentent plus de 5% de cette superficie, et de préférence plus de 10%.

## Revendications

1. Dispositif pour l'examen de la partie antérieure de l'œil par rétro-illumination de ladite partie antérieure de l'œil, comportant des moyens d'illumination de l'œil comprenant une source de lumière (1) configurés pour émettre un faisceau lumineux et des moyens d'imagerie de la partie antérieure de l'œil comprenant un capteur optique configuré pour capturer une image du segment antérieur de l'œil, **caractérisé en ce que** le dispositif comprend en outre des moyens de modulation configurés pour éteindre au moins une zone (ZE) du faisceau lumineux (FL1) émis depuis la source lumineuse (1) et pour créer ainsi au moins une zone non illuminée (ZNI) à la surface de l'œil.

2. Dispositif selon la revendication 1, **caractérisé en ce que** les moyens d'illumination comprennent une source de lumière (1) du type diode électroluminescente (DEL), des moyens de collimation pour former un faisceau lumineux, un diaphragme (3), des moyens de polarisation, des moyens de réflexion du faisceau lumineux vers l'œil d'un patient, et un moyen d'obturation d'une partie du faisceau lumineux formé, constituant les moyens de modulation d'une zone du faisceau lumineux.

3. Dispositif selon la revendication 2, **caractérisé en ce que** le moyen d'obturation est constitué par un disque opaque (4).

4. Dispositif selon l'une des revendications 2 et 3, **caractérisé en ce que** les moyens d'illumination comprennent au moins un miroir plan (M1) interposé entre le diaphragme (3) et les moyens de réflexion.

5. Dispositif selon la revendication 1, **caractérisé en ce que** les moyens d'illumination comprennent une source de lumière (1) du type diode électroluminescente (DEL), des moyens de collimation pour former un faisceau lumineux, un diaphragme (3), des moyens de polarisation, des moyens de réflexion du faisceau lumineux vers l'œil du patient, et un miroir (MP1) présentant au moins un orifice ne renvoyant pas au moins une partie du faisceau lumineux émis depuis la source (1) et constituant les moyens de modulation d'une zone du faisceau lumineux.

6. Dispositif selon la revendication 5, **caractérisé en ce que** le miroir (MP1) est parabolique et constitue également les moyens de collimation du faisceau lumineux.

7. Dispositif selon l'une des revendications 5 ou 6, **caractérisé en ce que** les moyens d'illumination comprennent une deuxième source lumineuse(1a), positionnée à l'arrière du miroir (MP1) et émettant un faisceau lumineux au travers de l'orifice dudit premier miroir (MP1), le faisceau lumineux issu de cette source lumineuse (1a) étant d'une longueur d'onde différente de celle du faisceau lumineux émis par la première source lumineuse (1) et formant un faisceau lumineux dit de fixation.

8. Dispositif selon la revendication 7, **caractérisé en ce que** les moyens d'imagerie comprennent un filtre spectral (80) destiné à bloquer le reflet du faisceau lumineux de fixation.

9. Dispositif selon l'une des revendications 5 à 7, **caractérisé en ce que** les moyens d'illumination comprennent un second miroir (MP2) interposé entre le diaphragme (3) et les moyens de réflexion.

10. Dispositif selon la revendication 1, **caractérisé en ce que** les moyens d'illumination comprennent une source de lumière tel que diode électroluminescente (DEL), des moyens de collimation pour former un faisceau lumineux, des moyens de polarisation et des moyens de réflexion pour projeter le faisceau lumineux vers l'œil d'un patient, des moyens de modulation spatiale de lumière étant prévus pour constituer des moyens de modulation d'au moins une zone du faisceau lumineux.

11. Dispositif selon l'une des revendications 2 à 10, **caractérisé en ce que** les moyens de polarisation sont constitués d'un cube polarisant qui remplace également les moyens de réflexion.

12. Dispositif selon l'une des revendications 1 à 11, **caractérisé en ce que** les moyens d'illumination et des moyens d'imagerie, sont sous forme de deux modules distincts.

13. Dispositif selon l'une quelconque des revendications précédentes, dans lequel les moyens d'imagerie sont configurés pour que le capteur d'image comprenne dans son champ de vision la zone non illuminée (ZNI) et au moins une zone illuminée (ZI) par le faisceau lumineux à la surface de l'œil.

14. Dispositif selon l'une quelconque des revendications précédentes, dans lequel les moyens de modulation sont configurés pour qu'une zone éteinte (ZE) soit entourée par une zone non éteinte (ZNE) dans le faisceau lumineux.

15. Dispositif selon l'une quelconque des revendications précédentes, dans lequel les moyens d'imagerie sont configurés pour présenter un champ de vision couvrant une surface d'un diamètre supérieur ou égal à 4 mm sur l'œil (O).

## Patentansprüche

1. Vorrichtung zur Untersuchung des vorderen Abschnitts des Auges durch Hintergrundbeleuchtung des vorderen Abschnitts des Auges, aufweisend Beleuchtungsmittel für das Auge, die eine Lichtquelle (1) umfassen, die ausgelegt sind, um einen Lichtstrahl zu senden, und Bildgebungsmittel des vorderen Abschnitts des Auges, die einen optischen Sensor umfassen, der ausgelegt ist, um ein Bild des vorderen Segments des Auges aufzunehmen, **dadurch gekennzeichnet, dass** die Vorrichtung ferner Modulationsmittel umfasst, die ausgelegt sind, um mindestens einen Bereich (ZE) des von der Lichtquelle (1) gesendeten Lichtstrahls (FL1) ausschalten und um so mindestens einen unbeleuchteten Bereich (ZNI) auf der Oberfläche des Auges zu erzeugen.

2. Vorrichtung nach Anspruch 1, **dadurch gekennzeichnet, dass** die Beleuchtungsmittel eine Lichtquelle (1) vom Typ Leuchtdiode (LED), Kollimationsmittel zum Bilden eines Lichtstrahls, eine Blende (3), Polarisationsmittel, Mittel zum Reflektieren des Lichtstrahls zum Auge eines Patienten und Mittel zum Verschließen eines Teils des gebildeten Lichtstrahls umfassen, die die Modulationsmittel eines Bereichs des Lichtstrahls bilden.

3. Vorrichtung nach Anspruch 2, **dadurch gekennzeichnet, dass** das Verschlussmittel aus einer undurchsichtigen Scheibe (4) besteht.

4. Vorrichtung nach einem der Ansprüche 2 und 3, **dadurch gekennzeichnet, dass** die Beleuchtungsmittel mindestens einen zwischen der Blende (3) und den Reflexionsmitteln angeordneten Planspiegel (M1) umfassen.

5. Vorrichtung nach Anspruch 1, **dadurch gekennzeichnet, dass** die Beleuchtungsmittel eine Lichtquelle (1) vom Typ Leuchtdiode (LED), Kollimationsmittel zum Bilden eines Lichtstrahls, eine Blende (3), Polarisationsmittel, Mittel zum Reflektieren des Lichtstrahls zum Auge des Patienten und einen Spiegel (MP1) umfassen, der mindestens einer Öffnung aufweist, die mindestens einen Teil des von der Quelle (1) gesendeten Lichtstrahls nicht reflektiert und die Modulationsmittel eines Bereichs des Lichtstrahls bildet.

6. Vorrichtung nach Anspruch 5, **dadurch gekennzeichnet, dass** der Spiegel (MP1) parabolisch ist und ebenfalls die Kollimationsmittel des Lichtstrahls bildet.

7. Vorrichtung nach einem der Ansprüche 5 oder 6, **dadurch gekennzeichnet, dass** die Beleuchtungsmittel eine zweite Lichtquelle (1a) umfassen, die hinter dem Spiegel (MP1) positioniert ist und einen Lichtstrahl durch die Öffnung des ersten Spiegels (MP1) sendet, wobei der von dieser Lichtquelle (1a) ausgehende Lichtstrahl eine andere Wellenlänge als der von der ersten Lichtquelle (1) gesendete Lichtstrahl hat und einen sogenannten Fixierungslichtstrahl bildet.

8. Vorrichtung nach Anspruch 7, **dadurch gekennzeichnet, dass** die Bildgebungsmittel einen Spektralfilter (80) umfassen, der dazu bestimmt ist, die Reflexion des Fixierungslichtstrahls zu blockieren.

9. Vorrichtung nach einem der Ansprüche 5 bis 7, **dadurch gekennzeichnet, dass** die Beleuchtungsmittel einen zweiten Spiegel (MP2) umfassen, der zwischen der Blende (3) und den Reflexionsmitteln angeordnet ist.

10. Vorrichtung nach Anspruch 1, **dadurch gekennzeichnet, dass** die Beleuchtungsmittel eine Lichtquelle wie beispielsweise eine Leuchtdiode (LED), Kollimationsmittel zum Bilden eines Lichtstrahls, Polarisationsmittel und Reflexionsmittel zum Projizieren des Lichtstrahls auf das Auge eines Patienten umfassen, wobei Mittel zur räumlichen Modulation des Lichts vorgesehen sind, um Modulationsmittel mindestens eines Bereichs des Lichtstrahls zu bilden.

11. Vorrichtung nach einem der Ansprüche 2 bis 10, **dadurch gekennzeichnet, dass** die Polarisationsmittel aus einem Polarisationswürfel bestehen, der ebenfalls die Reflexionsmittel ersetzt.

12. Vorrichtung nach einem der Ansprüche 1 bis 11, **dadurch gekennzeichnet, dass** die Beleuchtungsmittel und die Bildgebungsmittel in Form von zwei unterschiedlichen Modulen vorliegen.

13. Vorrichtung nach einem der vorstehenden Ansprüche, wobei die Bildgebungsmittel ausgelegt sind, damit der Bildsensor in seinem Sichtfeld den unbeleuchteten Bereich (ZNI) und mindestens einen durch den Lichtstrahl auf der Oberfläche des Auges beleuchteten Bereich (ZI) umfasst.

14. Vorrichtung nach einem der vorstehenden Ansprüche, wobei die Modulationsmittel ausgelegt sind, damit ein ausgeschalteter Bereich (ZE) von einem nicht ausgeschalteten Bereich (ZNE) im Lichtstrahl umgeben ist.

15. Vorrichtung nach einem der vorstehenden Ansprüche, wobei die Bildgebungsmittel ausgelegt sind, um ein Sichtfeld aufzuweisen, das eine Oberfläche mit einem Durchmesser von über oder gleich 4 mm auf dem Auge (O) abdeckt.

## Claims

1. A device for examining the anterior part of the eye by retroillumination of said anterior part of the eye, comprising means for illuminating the eye having a light source (1) configured to emit a light beam, and means for imaging the anterior part of the eye comprising an optical sensor configured to capture an image of the anterior segment of the eye, **characterized in that** the device further comprises modulating means configured to extinguish at least one region (ZE) of the light beam (FL1) emitted from the light source (1) and thus to create at least one non-illuminated region (ZNI) on the surface of the eye.

2. The device according to claim 1, **characterized in that** the illuminating means comprises a light source (1) of the light-emitting diode (LED) type, collimating means for forming a light beam, a diaphragm (3), polarizing means, means for reflecting the light beam toward the eye of a patient, and means for shutting off part of the light beam formed, constituting the means for modulating a region of the light beam.

3. The device according to claim 2, **characterized in that** the closure means is composed of an opaque disc (4).

4. The device according to one of claims 2 and 3, **characterized in that** the illuminating means comprises at least one plane mirror (M1) interposed between the diaphragm (3) and the reflecting means.

5. The device according to claim 1, **characterized in that** the illuminating means comprises a light source (1) of the light-emitting diode (LED) type, collimating means for forming a light beam, a diaphragm (3), polarizing means, means for reflecting the light beam toward the eye of the patient and a mirror (MP1) having at least one orifice not reflecting at least part of the light beam emitted from the source (1) and constituting means for modulating a region of the light beam.

6. The device according to claim 5, **characterized in that** the mirror (MP1) is parabolic and also constitutes the means for collimating the light beam.

7. The device according to one of claims 5 or 6, **characterized in that** the illuminating means comprises a second light source (1a), positioned behind the first mirror (MP1) and emitting a light beam through the orifice of said first mirror(MP1), the light beam coming from this light source (1a) being of a wavelength different from that of the light beam emitted by the first light source (1) and forming a light beam called a fixation light beam.

8. The device according to claim 7, **characterized in that** the imaging means comprises a spectral filter (80) intended to block the reflection of the fixation light beam.

9. The device according to one of claims 5 to 7, **characterized in that** the illuminating means comprises at least one second mirror (MP2) interposed between the diaphragm (3) and the reflecting means.

10. The device according to claim 1, **characterized in that** the illuminating means comprises a light source such as a light-emitting diode (LED), collimating means for forming a light beam, polarizing means, means for reflecting the light beam toward the eye of a patient, means for spatial modulation of the light being provided to constitute means for modulating a region of the light beam.

11. The device according to any of claims 2 to 10, **characterized in that** the polarizing means is composed of a polarizing cube that also replaces the reflecting means.

12. The device according to one of claims 1 to 11, **characterized in that** the illuminating means and the imaging means are in the form of two distinct modules.

13. The device according to any of the preceding claims, wherein the imaging means is configured so that the image sensor comprises in its field of view the non-illuminated region (ZNI) and at least one region (ZI) illuminated by the light beam on the surface of the eye.

14. The device according to any of the preceding claims, wherein the modulation means is configured so that an extinguished region (ZE) is surrounded by a non-extinguished region (ZNE) in the light beam.

15. The device according to any of the preceding claims, wherein the imaging means is configured to present a field of view covering an area of diameter greater than or equal to 4 mm on the eye (O).
